# EUROPEAN PATENT APPLICATION

(11) **EP 4 464 718 A1**
(43) Date of publication of application: **20.11.2024**
(21) Application number: 23767098.9
(22) Date of filing: 06.03.2023
(51) Int. Cl.: C07K 14/245, C12N 15/77, C12P 13/08

(54) **MUTANT L-THREONINE-RELEASING PROTEIN AND METHOD FOR PRODUCING L-THREONINE USING SAME**

(30) Priority: 07.03.2022 KR 20220028809
(71) Applicant: CJ CheilJedang Corporation, Seoul 04560 (KR)
(72) Inventor: CHOI, Woosung, Seoul 04560 (KR); JANG, Jaewon, Seoul 04560 (KR); BAEK, Mina, Seoul 04560 (KR); CHEONG, Ki Yong, Seoul 04560 (KR)
(74) Representative: Meissner Bolte Partnerschaft mbB
(86) International application number: PCT/KR2023/003005
(87) International publication number: WO 2023/171997

(57) **Abstract**

The present disclosure relates to an L-threonine efflux protein variant and a method for producing L-threonine using the same.

## Description

### [Technical Field]

The present disclosure relates to an L-threonine efflux protein variant and a method for producing L-threonine using the same.

### [Background Art]

Microorganisms of the genus *Corynebacterium* are Gram-positive microorganisms that are widely used for the production of L-amino acids. Among L-amino acids, L-threonine in particular is one of the essential amino acids and is used in the animal feed, human medicine, and cosmetics industries and is produced by fermentation using *Corynebacterium* strains.

Many attempts have been made to improve the method of producing L-amino acids using *Corynebacterium* strains. Among them, studies have been conducted to improve *Corynebacterium* strains that produce L-amino acids by destroying or attenuating a specific gene using recombinant DNA technology. Additionally, many attempts have been made to study the effect of amplification of the genes related to biosynthesis of each L-amino acid on L-amino acid production and to improve L-amino acid-producing *Corynebacterium* strains.

Additionally, in some cases, foreign genes derived from other bacteria may be introduced. However, there is still a need for the development of strains with improved L-threonine productivity even if the method follows the conventional methods described above.

Meanwhile, the expression of a specific amino acid efflux gene has contributed to improved productivity of the corresponding amino acid in microorganisms. Enhanced expression of the L-lysine efflux gene (*lysE*) in microorganisms of the genus *Corynebacterium* has led to the improvement of lysine productivity (WO 1997-023597). Additionally, EP 1016710 B1 discloses that the productivity of L-lysine, L-alanine, L-glutamic acid, L-threonine, L-histidine, L-valine, L-arginine, L-proline, and L-isoleucine was improved by enhancing *E*. *coli* genes whose functions have not been identified (*i.e*., *yeaS*, *yahN, yfiK,* and *yggA* genes).

### [Disclosure]

### [Technical Problem]

The present disclosure provides an L-threonine efflux protein variant in which the amino acid corresponding to the 133rd position in the amino acid sequence of SEQ ID NO: 1 is substituted with another amino acid.

The present disclosure provides a polynucleotide encoding the L-threonine efflux protein variant of the present disclosure.

The present disclosure provides a microorganism, which includes the L-threonine efflux protein variant of the present disclosure; or a polynucleotide encoding the same.

The present disclosure provides a method for producing L-threonine, which includes culturing a microorganism, which includes the L-threonine efflux protein variant of the present disclosure; or a polynucleotide encoding the same in a culture medium.

The present disclosure provides a composition for producing L-threonine, comprising the L-threonine efflux protein variant; a polynucleotide encoding the same; a vector comprising the polynucleotide; a microorganism comprising the L-threonine efflux protein variant or the polynucleotide encoding the same; a culture in which the microorganism has been cultured; or a combination of two or more thereof.

The present disclosure provides use of the L-threonine efflux protein variant; a polynucleotide encoding the same; a vector including the polynucleotide; or a microorganism comprising the L-threonine efflux protein variant or the polynucleotide encoding the same, for producing L-threonine.

### [Advantageous Effects]

When an L-threonine-producing microorganism is cultured using the L-threonine efflux protein variant of the present disclosure, it is possible to produce L-threonine with a higher yield compared to the microorganisms with the existing wild-type efflux protein.

### [Best Mode]

The present disclosure is described in detail as follows. Meanwhile, respective descriptions and embodiments disclosed in the present disclosure may also be applied to other descriptions and embodiments. That is, all combinations of various elements disclosed in the present disclosure fall within the scope of the present disclosure. Additionally, the scope of the present disclosure is not limited by the specific description below. Additionally, a number of papers and patent documents are referenced throughout this specification, and their citations are indicated. The contents of the cited documents and patent documents are incorporated by reference into this specification as a whole, and the level of the technical field to which the present disclosure belongs and the content of the present disclosure are more clearly described.

An aspect of the present disclosure provides an L-threonine efflux protein variant in which the amino acid corresponding to the 133rd position in the amino acid sequence of SEQ ID NO: 1 is substituted with another amino acid.

The L-threonine efflux protein variant of the present disclosure may be one in which the amino acid corresponding to the 133rd position of SEQ ID NO: 1 is substituted with an amino acid different from the amino acid before substitution. Alternatively, the L-threonine efflux protein variant may be an L-threonine efflux protein variant having a polar or hydrophilic amino acid among uncharged amino acids, in which the substituted amino acid is different from the amino acid before substitution, but is not limited thereto.

Specifically, the L-threonine efflux protein variant may be one in which the amino acid corresponding to the 133rd position in the amino acid sequence of SEQ ID NO: 1 is substituted with an amino acid selected from the group consisting of arginine, lysine, histidine, aspartic acid, asparagine, alanine, valine, leucine, isoleucine, methionine, phenylalanine, tryptophan, proline, serine, threonine, cysteine, tyrosine, glutamic acid, and glutamine, but is not limited thereto.

More specifically, the L-threonine efflux protein variant may be one in which the amino acid corresponding to the 133rd position in the amino acid sequence of SEQ ID NO: 1 is substituted with an amino acid selected from the group consisting of serine, threonine, cysteine, tyrosine, asparagine, and glutamine, for example, serine, but is not limited thereto.

Even more specifically, the L-threonine efflux protein variant of the present disclosure may have, include, consist of, or consist essentially of the amino acid sequence set forth in SEQ ID NO: 3.

The subject protein of the present disclosure, into which a mutation is to be introduced, may be a protein having L-threonine efflux activity. Specifically, the protein may be one which includes the amino acid sequence of SEQ ID NO: 1 and has threonine efflux activity, but is not limited thereto. The amino acid sequence does not exclude a meaningless sequence addition upstream or downstream of SEQ ID NO: 1, or a naturally occurring mutation therein, or a silent mutation therein, and as long as the protein having such mutation has an activity the same as or corresponding to that of the protein which includes the amino acid sequence of the corresponding SEQ ID NO: 1, it can correspond to the protein in which a mutation is to be introduced. For example, the subject protein of the present disclosure, into which a mutation is to be introduced, may be a protein consisting of an amino acid sequence having homology or identity of at least 80%, at least 85%, at least 90%, at least 95%, at least 96%, at least 97%, at least 98%, or at least 99% to the amino acid sequence of SEQ ID NO: 1. Additionally, it is apparent that any protein having an amino acid sequence with deletion, modification, substitution, or addition in a part thereof may also fall within the scope of the proteins of the present disclosure, into which a mutation is to be introduced, as long as the amino acid sequence has such homology or identity and shows an effect corresponding to the protein above.

The L-threonine efflux protein variant of the present disclosure may include an amino acid sequence, in which the amino acid corresponding to the 133rd position is an amino acid other than glycine (e.g., serine) based on the amino acid sequence of SEQ ID NO: 1, and which has homology or identity of at least 70%, at least 75%, at least 80%, at least 85%, at least 90%, at least 95%, at least 96%, at least 97%, at least 98%, at least 99%, or at least 99.5%, and less than 100% to the amino acid sequence of SEQ ID NO: 1. Additionally, it is apparent that any L-threonine efflux protein variant having an amino acid sequence with deletion, modification, substitution, or addition in a part thereof may also fall within the scope of the present disclosure as long as the amino acid sequence has such homology or identity and shows an effect corresponding to the protein above.

For example, the cases include the following: addition or deletion of a sequence to the N-terminus, C-terminus, and/or within the amino acid sequence that do not alter the function of the L-threonine efflux protein variant of the present disclosure, or a naturally occurring mutation, a silent mutation, or conservative substitution therein.

As used herein, the term "conservative substitution" refers to the substitution of one amino acid with another amino acid having similar structural and/or chemical properties. Such amino acid substitution may generally occur based on similarities in polarity, charge, solubility, hydrophobicity, hydrophilicity, and/or amphipathic nature of residues. Conventionally, conservative substitution may have little or no effect on the activity of a given protein or polypeptide.

For example, the amino acids may be classified as follows: among the electrically charged amino acids, positively charged (basic) amino acids include arginine, lysine, and histidine; and negatively charged (acidic) amino acids include glutamic acid and aspartic acid; and among the uncharged amino acids having an uncharged side chain include glycine, alanine, valine, leucine, isoleucine, methionine, phenylalanine, tryptophan, proline, serine, threonine, cysteine, tyrosine, asparagine, and glutamine.

As used herein, the term "protein variant" or "variant" refers to a polypeptide, in which one or more amino acids in an amino acid sequence of the polypeptide were subjected to conservative substitution and/or modification to thereby afford a difference in the amino acid sequence from that of the variant before mutation but capable of maintaining the functions or properties of the polypeptide. Such variants can generally be identified by modifying one or more amino acids in an amino acid sequence of the polypeptide and evaluating the properties of the modified polypeptide. That is, the ability of the variant may be increased, unchanged, or reduced compared to the polypeptide before the mutation. Additionally, some variants may include those in which at least one part (e.g., an N-terminal leader sequence or a transmembrane domain) is removed. Other variants may include those in which a part of the N-terminus and/or C-terminus of a mature protein is removed. The term "protein variant" may also be used interchangeably with "modification", "modified polypeptide", "modified protein", "mutant", "mutein", "divergent", *etc.,* and any term that is used in a sense of being mutated can be used without limitation thereto. For the purpose of the present disclosure, the variant may be a polypeptide in which the amino acid corresponding to the 133rd position in the amino acid sequence of SEQ ID NO: 1 is substituted with serine. Specifically, the variant may be a polypeptide including an amino acid sequence set forth in SEQ ID NO: 3, but is not limited thereto.

In addition, a variant may include deletions or additions of amino acids that have a minimal effect on the properties and secondary structure of a polypeptide. For example, a signal (or leader) sequence of a protein N-terminus involved in the cotranslational or post-translational transfer of proteins may be conjugated to the N-terminus of a variant. Additionally, the variant may also be conjugated to another sequence or linker so as to be identified, purified, or synthesized.

As used herein, the term "homology" or "identity" refers to a degree of relatedness between two given amino acid sequences or nucleotide sequences, and it may be expressed as a percentage. The terms homology and identity may often be used interchangeably.

The sequence homology or identity of conserved polynucleotides or polypeptides may be determined using a standard alignment algorithm, and default gap penalties established by a program to be used may be used together. Substantially, homologous or identical sequences can generally hybridize with each other along the entirety or a part of the sequences under moderate or highly stringent conditions. In hybridization, it is apparent that hybridizations between polynucleotides containing general codons or codons considering codon degeneracy are also included.

Whether any two polynucleotide- or polypeptide sequences have homology, similarity, or identity may be determined using a known computer algorithm such as the "FASTA" program using default parameters, for example, Pearson et al. (1988) Proc. Natl. Acad. Sci. USA 85:2444. Alternatively, this may be determined using the Needleman-Wunsch algorithm (Needleman and Wunsch, 1970, J. Mol. Biol. 48:443-453), which is performed in the Needleman program of the European Molecular Biology Open Software Suite (EMBOSS) package (Rice et al., 2000, Trends Genet. 16:276-277) (version 5.0.0 or later) (GCG program package (including GCG program package (Devereux, J. et al., Nucleic Acids Research 12:387 (1984)), BLASTP, BLASTN, FASTA (Atschul, S. F. et al., J Molec Biol 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST from the National Center for Biotechnology Information database or ClustalW.

The homology, similarity, or identity of polynucleotides or polypeptides may be determined, for example, by comparing sequence information using the GAP computer program (*e.g*., Needleman et al., (1970), J Mol Biol. 48:443) as disclosed in Smith and Waterman, Adv. Appl. Math (1981) 2:482. Briefly, the GAP program defines the homology, similarity, or identity as the value obtained by dividing the number of similarly aligned symbols (*i.e*., nucleotides or amino acids) by the total number of the symbols in the shorter of the two sequences. The default parameters for the GAP program may include: (1) unitary matrix (containing a value 1 for identity and a value 0 for non-identity) and the weighted comparison matrix of Gribskov et al. (1986) Nucl. Acids Res. 14:6745 as disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

In an embodiment of the present disclosure, the L-threonine efflux protein variant of the present disclosure may have an activity having increased L-threonine productivity compared to a wild-type polypeptide which has the activity of an L-threonine efflux protein.

As used herein, the term "L-threonine efflux protein (L-threonine exporter variant)" refers to a membrane protein having L-threonine efflux activity, and may be used interchangeably with RhtC. The amino acid sequence of RhtC can be obtained from NCBI GenBank, which is a known database, *etc.*

In an embodiment, the RhtC of the present disclosure may be derived from a microorganism, specifically a eukaryotic microorganism, and more specifically *Escherichia coli* (*E. coli*), but it may be a protein having L-threonine efflux activity derived from various kinds of microorganisms. In the amino acid sequence of the RhtC to be mutated in the present disclosure before modification, the amino acid corresponding to the 133rd position of SEQ ID NO: 1 before modification may be glycine (G).

As used herein, the term "corresponding to" refers to an amino acid residue at a recited position in a polypeptide, or an amino acid residue similar, identical, or homologous to a recited residue in a polypeptide. The identification of the amino acid at the corresponding position may be determining the specific amino acid in the sequence that refers to the specific sequence. As used herein, the term "corresponding region" generally refers to a similar or corresponding position in a related or reference protein.

For example, the numbering may be performed such that any amino acid sequence is aligned with SEQ ID NO: 1, and based on the result, each amino acid residue in the amino acid sequence is numbered by referring to the numerical position of the amino acid residue corresponding to the amino acid residue of SEQ ID NO: 1. For example, the sequence alignment algorithm, such as that described in the present disclosure, can identify the position of an amino acid, or the position where modification (e.g., substitution, insertion, deletion, *etc.)* occurs by comparing with a query sequence (also referred to as a "reference sequence").

For such alignment, for example, the Needleman-Wunsch algorithm (Needleman and Wunsch, 1970, J. Mol. Biol. 48:443-453), Needleman program of the European Molecular Biology Open Software Suite (EMBOSS) package (Rice et al., 2000, Trends Genet. 16:276-277), *etc.* may be used, but the program is not limited thereto, and any sequence alignment program known in the art, a pairwise sequence comparison algorithm, *etc.* may be appropriately used.

Another aspect of the present disclosure provides a polynucleotide encoding the L-threonine efflux protein variant of the present disclosure.

The *rhtC* gene of the present disclosure may include all of the genes known to encode proteins having RhtC activity.

Specifically, the *rhtC* gene of the present disclosure may be an *E. coli*-derived *rhtC* gene. In an embodiment, the *rhtC* gene of the present disclosure may be a polynucleotide encoding WP_000928824.1 derived from *E. coli.*

As used herein, the term "polynucleotide" refers to a polymer of nucleotide units linked in a chain type through covalent linkage, and to a DNA or RNA strand having a predetermined length or longer, and more specifically, to a polynucleotide fragment encoding the L-threonine efflux protein variant.

The polynucleotide encoding the L-threonine efflux protein variant of the present disclosure may include a nucleotide sequence encoding an L-threonine efflux protein variant, in which the amino acid corresponding to the 133rd position of SEQ ID NO: 1 is substituted with another amino acid; or a nucleotide sequence, in which the codon corresponding to the 397th to the 399th positions in the nucleotide sequence of SEQ ID NO: 2 is substituted with a codon encoding a different amino acid. Specifically, the polynucleotide of the present disclosure may include a nucleotide sequence encoding an amino acid sequence set forth in SEQ ID NO: 3. In a more specific embodiment of the present disclosure, the polynucleotide of the present disclosure may have or include the sequence of SEQ ID NO: 4 or SEQ ID NO: 5. Additionally, the polynucleotide of the present disclosure may consist of or essentially consist of the sequence SEQ ID NO: 4 or SEQ ID NO: 5.

The polynucleotide of the present disclosure may be variously modified in a coding region thereof within the range in which the amino acid sequence of the L-threonine efflux protein variant is not altered, considering codon degeneracy or the codons preferred by an organism in which the L-threonine efflux protein variant of the present disclosure is to be expressed. Specifically, the polynucleotide of the present disclosure may have or include a nucleotide sequence having homology or identity of at least 70%, at least 75%, at least 80%, at least 85%, at least 90%, at least 95%, at least 96%, at least 97%, at least 98%, and less than 100% to the sequence of SEQ ID NO: 2; may consist of or essentially consist of a nucleotide sequence having homology or identity of at least 70%, at least 75%, at least 80%, at least 85%, at least 90%, at least 95%, at least 96%, at least 97%, at least 98%, and less than 100% to the sequence of SEQ ID NO: 2, but the polynucleotide of the present disclosure is not limited thereto. In particular, in the sequence having such homologies or identities described above, the codon encoding the amino acid corresponding to the 133rd position of SEQ ID NO: 1 may be, for example, one of the codons encoding serine.

Additionally, with regard to the polynucleotide of the present disclosure, any sequence may be included without limitation as long as the sequence can hybridize with a probe that may be prepared from a known gene sequence (*e.g*., a complementary sequence to the entirety or a part of the polynucleotide sequence of the present disclosure) under stringent conditions. The "stringent conditions" refers to conditions that enable specific hybridization between polynucleotides. Such conditions are specifically described in literature (see Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York, 9.50-9.51, 11.7-11.8). For example, the conditions may include conditions under which polynucleotides having high homology or identity, such as genes having homology or identity of at least 70%, at least 75%, at least 80%, at least 85%, at least 90%, at least 95%, at least 96%, at least 97%, at least 98%, or at least 99%, but polynucleotides having lower homology or identity do not hybridize with each other; or typical washing conditions for Southern hybridization (*i.e*., washing once, specifically twice or three times at a salt concentration and temperature corresponding to 60°C, 1× SSC, and 0.1% SDS, specifically 60°C, 0.1× SSC, and 0.1% SDS, and more specifically 68°C, 0.1 × SSC, and 0.1% SDS.

Hybridization requires that two nucleic acids have complementary sequences, although mismatches between bases may be possible depending on hybridization stringency. The term "complementary" is used to describe the relationship between nucleotide bases that can hybridize with each another. For example, with respect to DNA, adenine is complementary to thymine, and cytosine is complementary to guanine. Therefore, the present disclosure may include not only substantially similar nucleic acid sequences, but also isolated nucleic acid fragments complementary to the entire sequence.

Specifically, polynucleotides having homology or identity with the polynucleotide of the present disclosure can be detected at a Tₘ value of 55°C using hybridization conditions that include a hybridization step and using the conditions described above. Additionally, the Tₘ value may be 60°C, 63°C, or 65°C, but is not limited thereto, and may be appropriately adjusted by those skilled in the art according to the purpose.

The appropriate stringency for hybridizing polynucleotides depends on the length of the polynucleotides and the degree of complementarity thereof, and variables thereof are well known in the art (*e.g*., Sambrook *et al., supra*).

Still another aspect of the present disclosure provides a vector including the polynucleotide of the present disclosure. The vector may be an expression vector for expressing the polynucleotide in a host cell, but is not limited thereto.

The vector of the present disclosure may include a DNA construct, which includes a nucleotide sequence of a polynucleotide encoding a target polypeptide and is operably linked to an appropriate expression control region (expression control sequence) so that the target polypeptide can be expressed in an appropriate host. The control sequence may include a promoter capable of initiating transcription, any operator sequence for controlling such transcription, a sequence for encoding an appropriate mRNA ribosome binding site, and sequences for controlling the termination of transcription and translation. The vector, after transformation into an appropriate host, may replicate or function irrespective of the genome of the host, or may be integrated into the genome itself.

The vector used in the present disclosure is not particularly limited, and any vector known in the art may be used. Examples of the vector commonly used may include native or recombinant plasmids, cosmids, viruses, and bacteriophages. For example, pWE15, M13, MBL3, MBL4, IXII, ASHII, APII, t10, t11, Charon4A, Charon21A, *etc.* may be used as phage vectors or cosmid vectors, and pDZ-based, pBR-based, pUC-based, pBluescriptII-based, pGEM-based, pTZ-based, pCL-based, pET-based vectors, *etc.* may be used as plasmid vectors. Specifically, pDZ, pDC, pDCM2, pACYC177, pACYC184, pCL, pECCG117, pUC19, pBR322, pMW118, pCC1BAC vectors, *etc.* may be used.

For example, a polynucleotide encoding a target polypeptide may be inserted into a chromosome through a vector for intracellular chromosomal insertion. The insertion of the polynucleotide into the chromosome may be performed using any method known in the art (*e.g*., homologous recombination), but is not limited thereto. The vector may further include a selection marker for identifying the insertion into the chromosome. A selection marker is used for selection of cells transformed with the vector, that is, to confirm whether the target nucleic acid molecule has been successfully inserted, and markers conferring selectable phenotypes (*e.g*., drug resistance, auxotrophy, resistance to cytotoxic drugs, expression of surface proteins, *etc.)* may be used. Under the circumstances where selective agents are treated, only the cells capable of expressing the selection markers can survive or express other phenotypic traits, so that the transformed cells can be selected.

As used herein, the term "transformation" means that a vector containing a polynucleotide encoding a target polypeptide is introduced into a host cell to express the polypeptide encoded by the polynucleotide in the host cell. As long as the polypeptide can be expressed in a host cell, it does not matter whether the polynucleotide is inserted and located on the chromosome of the host cell or located outside of the chromosome. Additionally, the polynucleotide includes DNA and/or RNA encoding the target polypeptide. As long as the polynucleotide can be introduced and expressed in the host cell, the polynucleotide may be introduced in any shape. For example, the polynucleotide may be introduced in the form of an expression cassette, which is a gene construct containing all factors required for self-expression. The expression cassette may include a promoter, a transcription termination signal, a ribosome binding site, and a translation termination signal, which may be operably linked to the polynucleotide. The expression cassette may be an expression vector enabling self-replication. Additionally, the polynucleotide may also be in the form introduced as-is into a host cell to be operably linked to the sequences required for expression in the host cell, but is not limited thereto.

Additionally, the term "operably linked" refers to a functional linkage between the polynucleotide sequence and a promoter sequence, which initiates and mediates the transcription of the polynucleotide encoding the desired L-threonine efflux protein variant of the present disclosure.

Still another aspect of the present disclosure provides a microorganism which includes the L-threonine efflux protein variant of the present disclosure or the polynucleotide of the present disclosure

The strain of the present disclosure may include the L-threonine efflux protein variant of the present disclosure, a polynucleotide encoding the polypeptide, or a vector including the polynucleotide of the present disclosure.

As used herein, the term "microorganism (or strain)" includes both wild-type microorganisms and the microorganisms in which genetic modification has occurred naturally or artificially, and it may refer to a microorganism, in which a specific mechanism has been attenuated or enhanced caused by the insertion of a foreign gene, enhancement/inactivation of an endogenous gene, *etc.* and which includes a genetic modification for the production of a desired polypeptide, protein, or product.

The strain of the present disclosure may be a strain naturally having L-threonine productivity or a microorganism obtained by imparting L-threonine productivity to a strain without L-threonine productivity. In an embodiment, the strain may be a microorganism having improved L-threonine efflux ability by the introduction of the L-threonine efflux protein variant of the present disclosure or a polynucleotide encoding the same, but the strain is not limited thereto.

The strain of the present disclosure may be a microorganism which has increased L-threonine productivity compared to its parent strain not including a variant of the present disclosure or a wild-type strain belonging to the genus *Corynebacterium.* The microorganism may be one having improved L-threonine productivity due to the increase of L-threonine efflux ability by the introduction of the variant of the present disclosure.

For example, the microorganism with an unmodified L-threonine efflux protein, which is a subject strain of the present disclosure to compare the presence/absence of an increase in L-threonine productivity, may be the *Corynebacterium glutamicum* CA09-0903 strain (Accession No. KCCM12502P, KR Patent No. 10-2126951), which is an L-threonine-producing strain) or the *Corynebacterium glutamicum* KFCC10881-THR strain (Accession No. KCCM11222P, U.S. Patent No. 10590446 B2), but the microorganism is not limited thereto.

In an embodiment, the recombinant strain having increased L-threonine productivity may be one which has increased L-threonine productivity of about at least 1%, specifically about at least 1%, about at least 2.5%, about at least 5%, about at least 7.5%, about at least 10%, about at least 15%, about at least 17.5%, about at least 20%, about at least 22.5%, about at least 25%, about at least 27.5%, about at least 30%, about at least 32.5%, or about at least 33% (the upper limit value is not particularly limited, and it may be, for example, about 200% or less, about 150% or less, about 100% or less, about 50% or less, about 40% or less, about 30% or less, about 20% or less, or about 15% or less) compared to the L-threonine productivity of the parent strain before modification or an unmodified microorganism, but the recombinant strain is not limited thereto as long as it has an increased amount of positive (+) value compared to the L-threonine productivity of the parent strain before modification, an unmodified microorganism, or a microorganism with an unmodified L-threonine efflux protein. In another embodiment, the recombinant strain having increased L-threonine productivity of about at least 1.01-fold, about at least 1.025-fold, about at least 1.075-fold, about at least 1.10-fold, about at least 1.125-fold, about at least 1.15-fold, about at least 1.175-fold, about at least 1.225-fold, about at least 1.25-fold, about at least 1.275-fold, about at least 1.30-fold, about at least 1.325-fold, or about at least 1.33-fold (the upper limit value is not particularly limited, and it may be, for example, about 10-fold or less, about 5-fold or less, about 3-fold or less, or about 2-fold or less) compared to the L-threonine productivity of the parent strain before modification or an unmodified microorganism, but the recombinant strain is not limited thereto.

As used herein, the term "unmodified microorganism" does not exclude strains including a mutation that may naturally occur in a microorganism, and may refer to a wild-type strain or natural-type strain itself or a strain before transformation caused by genetic mutation due to natural or artificial factors. Additionally, as used herein, the term "microorganism with an unmodified L-threonine efflux protein" may refer to a strain which is not introduced with the L-threonine efflux protein variant described herein or a strain before the introduction of the same. The microorganism with an unmodified L-threonine efflux protein does not exclude strains which include modifications of proteins or genes other than the modification of the L-threonine efflux protein or a polynucleotide encoding the same.

As used herein, the term "unmodified microorganism" may be used interchangeably with "strain before modification", "microorganism before modification", "unmutated strain", "unmodified strain", "unmodified microorganism" or "reference microorganism".

The microorganism of the present disclosure may be a microorganism including an L-threonine efflux protein variant or a polynucleotide encoding the same; alternatively, it may be a genetically modified microorganism (*e.g*., a recombinant microorganism) to include an L-threonine efflux protein variant or a polynucleotide encoding the same, but is not limited thereto. The "endogenous activity" refers to the activity of a particular polypeptide originally possessed by a parent strain before transformation thereof when a microorganism is transformed by genetic mutation caused by natural or artificial factors.

In another embodiment of the present disclosure, the microorganism of the present disclosure may be *Corynebacterium stationis, Corynebacterium glutamicum, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

In another embodiment, the recombinant microorganism of the present disclosure may be a microorganism in which the activity of some of the proteins in the L-threonine biosynthetic pathway is further enhanced or the activity of some of the proteins in the L-threonine degradation pathway is further weakened, thus affording enhanced L-threonine productivity.

Specifically, for the enhancement of the biosynthesis pathway of L-threonine in the microorganism of the present disclosure, for example, the expression of *thrC* gene encoding threonine synthase, *ppc* gene encoding phosphoenolpyruvate carboxylase, *galP* gene involved in glucose uptake, *lysC* gene encoding lysine-sensitive aspartokinase 3, *hom* gene encoding homoserine dehydrogenase, or *pyc* gene inducing an increase in oxaloacetate pool may be enhanced or increased in the microorganism.

For the release of the feedback inhibition for L-threonine, for example, a genetic mutation for the *lysC* gene, the *hom* gene, the *thrA* gene having bifunctional aspartokinase/homoserine dehydrogenase 1, etc. may be introduced into the microorganism.

For the inactivation of a gene attenuating the bio-synthesis pathway of L-threonine, for example, the expression of the *pckA* gene involved in the conversion of oxaloacetate (OAA), which is an intermediate in the L-threonine biosynthesis, into phosphoenolpyruvate (PEP); *tyrR* gene inhibiting the *lysC* gene; *galR* gene inhibiting the expression of the *galP* gene involved in glucose uptake, or *mcbR* gene, which is a DNA-binding transcriptional dual regulator, may be attenuated or inactivated in the microorganism.

For the increase of the activity of the L-threonine operon, a plasmid including a threonine operon composed of genes encoding aspartokinase, homoserine dehydrogenase, homoserine kinase, and threonine synthase (Japanese Patent Application Publication No. 2005-227977), an E. coli-derived threonine operon, *etc.* may be introduced into the microorganism (TURBA E, et al., Agric. Biol. Chem. 53:2269-2271, 1989), thereby increasing the expression of the threonine operon in the microorganism.

Additionally, resistance to α-amino-β-hydroxy valeric acid, D,L-threonine hydroxamate, which are L-threonine analogs, *etc.* may be imparted.

Additionally, *dapA* (dihydrodipicolinate synthase), *lysA* (diaminopimelate decarboxylase), and *ddh* (diaminopimelate dehydrogenase) genes, which act on the L-lysine biosynthesis pathway having common precursors as L-threonine may be weakened.

However, without limitation thereto, the L-threonine productivity can be enhanced by way of a gene expression control method known in the art.

As used herein, the "weakening" of the activity of a polypeptide (*e.g*., including the proteins specified by the name of each enzyme) is a concept that includes both reduced activity and no activity compared to endogenous activity. The weakening can be interchangeably used with terms such as inactivation, deficiency, down-regulation, decrease, reduction, and attenuation.

The weakening may also include a case where the activity of the polypeptide itself is reduced or removed compared to the activity of the polypeptide originally possessed by the microorganism due to a mutation of the polynucleotide encoding the polypeptide, *etc*.; a case where the overall polypeptide activity level and/or concentration (expression level) in the cell is lower than that of the natural strain due to inhibition of expression of a gene encoding the same or inhibition of translation into the polypeptide; a case where the expression of the polynucleotide is not achieved at all; and/or a case where even if the polynucleotide is expressed, it has no activity. The "inactivation, deficiency, reduction, down-regulation, reduction, and attenuation" of the activity of a polypeptide compared to its endogenous activity means that its activity had become lower than the activity of a specific polypeptide originally possessed by the parent strain or an unmodified microorganism before transformation.

Such weakening of the activity of a polypeptide may be performed by any method known in the art, but is not limited thereto, and may be achieved by applying various methods well known in the art (*e.g*., Nakashima N et al., Bacterial cellular engineering by genome editing and gene silencing. Int J Mol Sci. 2014; 15(2): 2773-2793; Sambrook et al. Molecular Cloning 2012, etc.).

Specifically, the weakening of the activity of the polypeptide of the present disclosure may be achieved by:
1) deletion of all or part of a gene encoding a polypeptide;
2) modification of an expression control region (or expression control sequence) so as to reduce the expression of a gene encoding a polypeptide;
3) modification of the amino acid sequence constituting a polypeptide (*e.g*., deletion/substitution/addition of one or more amino acids on the amino acid sequence) so as to remove or weaken the activity of the polypeptide;
4) modification of the sequence of a gene encoding the polypeptide so that the activity of the polypeptide is removed or weakened (*e.g*., deletion/substitution/addition of one or more nucleic acid bases on the nucleotide sequence of the polypeptide gene so as to encode a modified polypeptide such that the activity of the polypeptide is removed or weakened);
5) modification of the nucleotide sequence encoding the start codon or 5'-UTR region of a gene transcript encoding a polypeptide;
6) introduction of an antisense oligonucleotide (*e.g*., antisense RNA) complementary to the transcript of a gene encoding a polypeptide;
7) addition of a sequence complementary to the Shine-Dalgarno sequence at an upstream region of the Shine-Dalgarno sequence of a gene encoding a polypeptide so as to form a secondary structure to which ribosomes cannot attach;
8) addition of a promoter transcribed in the opposite direction to the 3' end of the open reading frame (ORF) of the sequence of a gene encoding a polypeptide (reverse transcription engineering, RTE); or
9) a combination of two or more selected from 1) to 8) above, but the present disclosure is not particularly limited thereto.

For example, the 1) deletion of all or part of a gene encoding a polypeptide may be removal of the entire polynucleotide encoding the endogenous target polypeptide in the chromosome, replacement with a polynucleotide in which some nucleotides are deleted, or replacement with a marker gene.

Additionally, the 2) modification of an expression control region (or expression control sequence) may be development of a mutation in the expression control region (or expression control sequence) by deletion, insertion, non-conservative or conservative substitution, or a combination thereof, or replacement with a sequence having weaker activity. The expression control region includes a promoter, an operator sequence, a sequence encoding a ribosome binding site, and sequences controlling the termination of transcription and translation, but the expression control region is not limited thereto.

The 3) modification of an amino acid sequence and the 4) modification of a polynucleotide sequence may be development of a mutation in the amino acid sequence of the polypeptide or the polynucleotide sequence encoding the polypeptide by deletion, insertion, non-conservative or conservative substitution, or a combination thereof so as to weaken the activity of the polypeptide; or replacement of the amino acid sequence or polynucleotide sequence with an improved amino acid sequence so as to have weaker activity or replacement of the amino acid sequence or polynucleotide sequence with an improved amino acid sequence so as to have no activity, but the 3) and 4) modifications are not limited thereto. For example, the expression of a gene may be inhibited or weakened by introducing a mutation in the polynucleotide sequence so as to form a stop codon, but is not limited thereto.

The 5) modification of a nucleotide sequence encoding the start codon or 5'-UTR region of a gene transcript encoding a polypeptide may be, for example, a substitution of the nucleotide sequence with a nucleotide sequence encoding another start codon having a lower expression rate of the polypeptide compared to the endogenous start codon, but is not limited thereto.

The 6) introduction of an antisense oligonucleotide (*e.g*., antisense RNA) complementary to the transcript of a gene encoding a polypeptide is disclosed in references, for example, [Weintraub, H. et al., Antisense-RNA as a molecular tool for genetic analysis, Reviews - Trends in Genetics, Vol. 1(1) 1986].

The 7) addition of a sequence complementary to the Shine-Dalgarno sequence at an upstream region of the Shine-Dalgarno sequence of a gene encoding a polypeptide so as to form a secondary structure to which ribosomes cannot attach may be to make mRNA translation impossible or slow down the mRNA translation rate.

Additionally, the 8) addition of a promoter transcribed in the opposite direction to the 3' end of the open reading frame (ORF) of the sequence of a gene encoding a polypeptide (reverse transcription engineering, RTE) may be to weaken the activity by making an antisense nucleotide sequence complementary to the transcript of the gene encoding the polypeptide.

As used herein, the term "enhancement" of the activity of a polypeptide means that the activity of a polypeptide is enhanced compared to the endogenous activity of the polypeptide. The enhancement may be used interchangeably with terms such as activation, up-regulation, overexpression, and increase. In particular, activation, enhancement, up-regulation, overexpression, and increase may include all of an exhibition of an activity that was not originally possessed, or an exhibition of an enhanced activity compared to its endogenous activity or activity before modification. "Enhancement", "up-regulation", "overexpression" or "increase" of the activity of a polypeptide compared to its endogenous activity means that the activity and/or concentration (expression level) is improved.

The enhancement may be achieved by introducing a foreign polypeptide or by enhancing the activity and/or concentration (expression level) of an endogenous polypeptide. Whether or not the activity of the polypeptide is enhanced can be confirmed from an increase in the activity level, expression level, or amount of a product released from the corresponding polypeptide.

The enhancement of the activity of the polypeptide can be achieved by various methods well known in the art, and is not limited as long as the activity of a target polypeptide can be enhanced compared to that of the microorganism before transformation. Specifically, the enhancement may be achieved using technologies in the fields of genetic engineering and/or protein engineering well known to those skilled in the art, which are routine methods of molecular biology, but the methods are not limited thereto (*e.g.*, Sitnicka et al. Functional Analysis of Genes. Advances in Cell Biology. 2010, Vol. 2. 1-16; Sambrook et al. Molecular Cloning 2012, *etc.).*

Specifically, the enhancement of a polypeptide may be achieved in the present disclosure by:
1) an increase in the intracellular copy number of a polynucleotide encoding a polypeptide;
2) replacement of the expression control region of a polypeptide-encoding gene on the chromosome with a sequence having strong activity;
3) modification of the nucleotide sequence encoding the start codon or 5'-UTR region of a gene transcript encoding a polypeptide;
4) modification of the amino acid sequence of a polypeptide so as to enhance the activity of the polypeptide;
5) modification of a polynucleotide sequence encoding a polypeptide so as to enhance the activity of the polypeptide (*e.g*., modification of the polynucleotide sequence of the polypeptide gene so as to encode a modified polypeptide that enhances the activity of the polypeptide);
6) introduction of a foreign polypeptide exhibiting the activity of a polypeptide or a foreign polynucleotide encoding the polypeptide;
7) codon optimization of a polynucleotide encoding a polypeptide;
8) analysis of the tertiary structure of a polypeptide, selection of exposed sites, and modification or chemical modification of the same; or
9) a combination of two or more selected from 1) to 8) above, but the present disclosure is not particularly limited thereto.

More specifically, the 1) increase in the intracellular copy number of a polynucleotide encoding a polypeptide may be achieved by introducing into a host cell a vector capable of replicating and functioning independently of the host, to which a polynucleotide encoding the polypeptide is operably linked. Alternatively, it may be achieved by introducing one copy or two or more copies of a polynucleotide encoding the corresponding polypeptide into the chromosome of the host cell. The introduction into the chromosome may be performed by introducing a vector, which is capable of inserting the polynucleotide into the chromosome of the host cell, into the host cell, but is not limited thereto. The vector is as described above.

The 2) replacement of the expression control region (or expression control sequence) of a polypeptide-encoding gene on the chromosome with a sequence having strong activity may be, for example, development of a mutation in the expression control region (or expression control sequence) by deletion, insertion, non-conservative or conservative substitution, or a combination thereof, or replacement with a sequence having weaker activity so as to enhance the activity of the expression control region. The expression control region may include a promoter, an operator sequence, a sequence encoding a ribosome-binding site, and a sequence controlling termination of transcription and translation, but is not particularly limited thereto.

Known examples of the strong promoter may include CJ1 to CJ7 promoters (U.S. Patent No. 7662943 B2), a lac promoter, a trp promoter, a trc promoter, a tac promoter, a lambda phage PR promoter, a PL promoter, a tet promoter, a gapA promoter, an SPL7 promoter, an SPL13 (sm3) promoter (U.S. Patent No. 10584338 B2), an O2 promoter (U.S. Patent No. 10273491 B2), a tkt promoter, a yccA promoter, *etc.,* but are not limited thereto.

The 3) modification of the nucleotide sequence encoding the start codon or 5'-UTR region of a gene transcript encoding a polypeptide may be, for example, a substitution of the nucleotide sequence with a nucleotide sequence encoding another start codon having a higher expression rate of the polypeptide compared to the endogenous start codon, but is not limited thereto.

The 4) modification of an amino acid sequence and the 5) modification of a polynucleotide sequence may be development of a mutation in the amino acid sequence of the polypeptide or the polynucleotide sequence encoding the polypeptide by deletion, insertion, non-conservative or conservative substitution, or a combination thereof so as to enhance the activity of the polypeptide; or replacement of the amino acid sequence or polynucleotide sequence with an improved amino acid sequence so as to have much stronger activity or replacement of the amino acid sequence or polynucleotide sequence with an improved amino acid sequence so as to have increased activity, but the 4) and 5) modifications are not limited thereto. The replacement may specifically be performed by inserting a polynucleotide into a chromosome by homologous recombination, but is not limited thereto. The vector used at this time may further include a selection marker to confirm whether or not the chromosome has been inserted.

The 6) introduction of a foreign polynucleotide exhibiting the activity of the polypeptide may be the introduction of a foreign polynucleotide encoding a polypeptide exhibiting an activity the same as/similar to that of the polypeptide into a host cell. The foreign polynucleotide is not limited in its origin or sequence as long as it exhibits an activity the same as/similar to that of the polypeptide. The method used for the introduction may be performed by appropriately selecting a transformation method known to those skilled in the art, and the polynucleotide introduced is expressed in the host cell to produce the polypeptide, thereby increasing its activity.

The 7) codon optimization of a polynucleotide encoding a polypeptide may be codon optimization of an endogenous polynucleotide so as to increase transcription or translation of the endogenous polynucleotide in a host cell or codon optimization of a foreign polynucleotide whose codons are optimized so as to achieve optimized transcription and translation of the foreign polynucleotide in a host cell.

The 8) analysis of the tertiary structure of a polypeptide, selection of exposed sites, and modification or chemical modification of the same may be, for example, determining a template protein candidate according to the degree of sequence similarity by comparing the sequence information of the polypeptide to be analyzed with the database where the sequence information of base proteins is stored, and confirming the structure based on the same, thereby modifying or chemically modifying the same.

Such enhancement of the activity of a polypeptide may be an increase of the activity, expression or concentration of the corresponding polypeptide based on the activity, concentration of the polypeptide expressed in a wild-type or unmodified microbial strain or an increase in the amount of the product produced from the polypeptide, but is not limited thereto.

The modification of the entirety or a part of a polynucleotide in the microorganism of the present disclosure may be induced by (a) homologous recombination using a vector for chromosomal insertion into a microorganism or genome editing using an engineered nuclease (*e.g*., CRISPR-Cas9) and/or (b) treatment with light (*e.g*., ultraviolet rays, radiation, *etc.)* and/or chemicals, but the modification method is not limited thereto. The method of modifying the entirety or a part of a gene may include a method by DNA recombination technology. For example, deletion of the entirety or a part of a gene may be achieved by injecting a nucleotide sequence or vector including a nucleotide sequence homologous to a target gene into the microorganism so as to cause homologous recombination. The injected nucleotide sequence or vector may include a dominant selection marker, but is not limited thereto.

In the microorganism of the present disclosure, the L-threonine efflux protein variant, polynucleotide, L-threonine, *etc.* are as described in other aspects above.

Still another aspect of the present disclosure provides a method for producing L-threonine, which includes culturing a microorganism including the L-threonine efflux protein variant of the present disclosure or the polynucleotide of the present disclosure in a medium.

The method for producing L-threonine of the present disclosure may include culturing a microorganism including the L-threonine efflux protein variant of the present disclosure, the polynucleotide of the present disclosure, or the vector of the present disclosure in a medium.

As used herein, the term "culture" refers to growing the microorganism of the present disclosure under appropriately adjusted environmental conditions. The culture procedure of the present disclosure may be performed according to appropriate media or culture conditions known in the art. Such a culture procedure may easily be adjusted according to the selected strain by those skilled in the art. Specifically, examples of the culture may include a batch culture, a continuous culture, and a fed-batch culture, but are not limited thereto.

As used herein, the "medium" refers to a mixture including as main ingredients nutrient substances required for the culture of the microorganism of the present disclosure, wherein the medium supplies nutrient substances, growth factors, *etc.,* including water, which are essential for survival and growth. Specifically, as for the media and other culture conditions used for culturing the microorganism of the present disclosure, any medium used for typical culture of microorganisms may be used without particular limitation, and the microorganism of the present disclosure may be cultured under aerobic conditions in a conventional medium including appropriate carbon sources, nitrogen sources, phosphorus sources, inorganic compounds, amino acids, and/or vitamins, while the temperature, pH, *etc.* are adjusted.

Specifically, for the culture media for microorganisms of the genus *Corynebacterium,* please refer to the literature ("Manual of Methods for General Bacteriology" by the American Society for Bacteriology (Washington D.C., USA, 1981)).

In the present disclosure, the carbon source may include carbohydrates (*e.g*., glucose, saccharose, lactose, fructose, sucrose, maltose, *etc*.); sugar alcohols (*e.g*., mannitol, sorbitol, *etc*.); organic acids (*e.g*., pyruvic acid, lactic acid, citric acid, *etc.);* amino acids (*e.g*., glutamic acid, methionine, lysine, *etc*.); *etc.* Additionally, natural organic nutrient sources (*e.g*., starch hydrolysates, molasses, blackstrap molasses, rice bran, cassava, bagasse, and corn steep liquor) may be used, and specifically, carbohydrates (*e.g*., glucose, sterilized pretreated molasses (*i.e*., molasses converted to reduced sugars), *etc*.) may be used, and appropriate amounts of other carbon sources may be used without limitation. These carbon sources may be used alone or in a combination of two or more, but are not limited thereto.

As for the nitrogen sources, inorganic nitrogen sources (*e.g*., ammonia, ammonium sulfate, ammonium chloride, ammonium acetate, ammonium phosphate, ammonium carbonate, ammonium nitrate, *etc*.); amino acids (*e.g*., glutamic acid, methionine, glutamine, *etc*.); and organic nitrogen sources (*e.g*., peptone, NZ-amine, meat extracts, yeast extracts, malt extracts, corn steep liquor, casein hydrolysates, fishes or decomposition products thereof, defatted soybean cake or decomposition products thereof, *etc*.) may be used. These nitrogen sources may be used alone or in a combination of two or more, but are not limited thereto.

The phosphate sources may include potassium phosphate monobasic, potassium phosphate dibasic, corresponding sodium-containing salts, *etc.* As for inorganic compounds, sodium chloride, calcium chloride, iron chloride, magnesium sulfate, iron sulfate, manganese sulfate, calcium carbonate, *etc.* may be used, and besides, amino acids, vitamins, and/or suitable precursors, *etc.* may be included. These sources or precursors may be added to the medium in a batch or continuous manner. However, the medium of the present disclosure is not limited thereto.

Additionally, the pH of the medium may be adjusted by adding compounds (*e.g*., ammonium hydroxide, potassium hydroxide, ammonia, phosphoric acid, sulfuric acid, *etc.)* to the medium in an appropriate manner during the culture of the microorganism. Additionally, a defoaming agent (*e.g*., a fatty acid polyglycol ester) may be added to prevent foam formation. Additionally, oxygen or oxygen-containing gas may be injected into the medium to maintain the aerobic state of the medium, or nitrogen, hydrogen, or carbon dioxide gas or no gas may be injected to maintain an anaerobic or microaerobic state of the medium, but is not limited thereto.

The temperature of the medium may be 20°C to 45°C, and specifically 25°C to 40°C, and specifically may be cultured for 10 to 160 hours, but the culture is not limited thereto.

L-Threonine produced by the culture of the present disclosure may be released into the medium or may remain in cells.

The method for producing L-threonine of the present disclosure may further include a step of preparing the microorganism of the present disclosure, a step of preparing a medium for culturing the microorganism, or a combination thereof (in any order), for example, prior to the culturing step.

The method for producing L-threonine of the present disclosure may further include a step of recovering L-threonine from the culture medium (a medium where the culture has been performed) or a *Corynebacterium glutamicum* strain according to the culture. The recovering step may be further included after the culturing step.

The recovery may be to collect desired L-threonine from the culture using an appropriate method known in the art according to the culturing method of microorganisms (*e.g*., batch, continuous, fed-batch culture methods, *etc*.). For example, centrifugation, filtration, treatment with a crystallized protein precipitating agent (salting-out method), extraction, ultrasonic disruption, ultrafiltration, dialysis, molecular sieve chromatography (gel filtration), various types of chromatography (*e.g*., adsorption chromatography, ionexchange chromatography, affinity chromatography, *etc.),* HPLC, and a combination of these methods may be used, and desired L-threonine can be recovered from the medium or microorganism by using an appropriate method known in the art.

Additionally, the method for producing L-threonine of the present disclosure may further include a purification step. The purification may be performed using any suitable method known in the art. In an embodiment, when the method for producing L-threonine of the present disclosure includes both the recovery step and the purification step, the recovery step and the purification step may be performed continuously or discontinuously regardless of order, or may be performed simultaneously or integrated into one step, but the method is not limited thereto.

In the method of the present disclosure, the L-threonine efflux protein variant, polynucleotide, vector, strain, *etc.* are as described in other aspects above.

Still another aspect of the present disclosure provides a composition for producing L-threonine, in which the composition comprises the L-threonine efflux protein variant of the present disclosure; a polynucleotide encoding the same; a vector comprising the polynucleotide; a microorganism comprising the L-threonine efflux protein variant or the polynucleotide encoding the same; a culture in which the microorganism has been cultured; or a combination of two or more thereof.

The composition of the present disclosure may further include any suitable excipients commonly used in compositions for producing L-amino acids, and such excipients may be, for example, preservatives, wetting agents, dispersing agents, suspending agents, buffers, stabilizing agents, tonicity agents, *etc.,* but are not limited thereto.

Still another aspect of the present disclosure provides a use of the L-threonine efflux protein variant of the present disclosure; a polynucleotide encoding the same; a vector including the polynucleotide; or a microorganism comprising the L-threonine efflux protein variant or the polynucleotide encoding the same, for producing L-threonine.

The L-threonine efflux protein, L-threonine efflux protein variant, polynucleotide, vector, strain, medium, L-threonine, *etc.* are as described in other aspects above.

### [Mode for Invention]

Hereinafter, the present disclosure will be described in detail through exemplary embodiments. However, these exemplary embodiments are for illustrative purposes only and are not intended to limit the scope of the present disclosure. Meanwhile, technical matters not described in this specification can be sufficiently understood and easily implemented by those skilled in the art of the present disclosure or similar technical fields.

### Example 1: Preparation of libraries and plasmids of L-threonine efflux protein variants

To prepare a template to be used in error-prone PCR, a PrhtC nucleotide sequence fragments (SEQ ID NO: 6) were obtained from the genomic DNA of *E. coli* W3110 by performing PCR using a primer pair of SEQ ID NO: 7 and SEQ ID NO: 8.

The primers used are shown in Table 1 below.

**[Table 1]**

| SEQ ID NO | Sequence Name | Sequence (5' -> 3') |
|---|---|---|
| 7 | PrhtC F | TCGAGCTCGGTACCCGGGGAGCCATGAACATTGGG TGAGC |
| 8 | PrhtC R | GTCGACTCTAGAGGAggatccACATTTGACTCGCGGG GGA |

Then, the pCC1BAC(EPICENTRE) digested with Smal restriction enzyme and the DNA fragments obtained above were cloned using the Gibson assembly method to obtain pCC1BAC-PrhtC as a recombinant plasmid. The cloning was performed by mixing the Gibson assembly reagent and each of the gene fragments in a calculated number of moles, followed by preservation at 50°C for 1 hour.

Error-prone PCR was performed on wild-type rhtC *(rhtC* WT) (SEQ ID NO: 2) encoding the L-threonine efflux protein so as to induce random mutagenesis using the diversify PCR random mutagenesis kit (Takara). For the selection of mutation rate conditions, error-prone PCR was performed according to the MnSO₄ concentration under the following two conditions. The genomic DNA of *E*. *coli* W3110 was used as a DNA template into which mutation was to be introduced. The composition of the reactants for error-prone PCR is shown in Table 2 below. The PCR was performed as follows: denaturation at 95°C for 30 seconds; 25 cycles of denaturation at 95°C for 30 seconds, annealing at 55°C for 30 seconds, and polymerization at 68°C for 30 seconds; and polymerization at 68°C for 60 seconds. The PCR was performed using a primer pair of SEQ ID NOS: 9 and 10.

The primers used are shown in Table 3 below.

**[Table 2]**

| Case # | 1 (µL) | 2 (µL) |
|---|---|---|
| 10× Titanium taq Buffer | 5 | 5 |
| MnSO₄ (8 mM) | 1 | 2 |
| dGTP (2 mM) | 1 | 1 |
| 50× dNTP Mix | 1 | 1 |
| Titanium Taq Polymerase | 1 | 1 |
| Forward primer (5 pmol) | 2 | 2 |
| Reverse primer (5 pmol) | 2 | 2 |
| Template DNA | 1 | 1 |
| dH₂O | 36 | 35 |
| Total | 50 | 50 |

**[Table 3]**

| SEQ ID NO | Sequence Name | Sequence (5' -> 3') |
|---|---|---|
| 9 | rhtC F | TCCCCCGCGAGTCAAATGTgATGTTGATGTTATTTCT CACCGTC |
| 10 | rhtC R | GTCGACTCTAGAGGAggatcTCACCGCGAAATAATCAA ATGAATG |

The products obtained by error-prone PCR performed under the conditions of Table 2 were treated with *Dpn*I, and then cloned into the pCC1BAC-PrhtC digested with BamHI restriction enzyme by the Gibson assembly method to obtain a library of recombinant mutant plasmid pCC1BAC-PrhtC_rhtC(mt). The thus-obtained mutant library was transformed into *E*. *coli* K12 cells, and the transformed cells were plated on LB plate media containing 50 µg/L spectinomycin.

Fifty colonies were selected from the library of *E*. *coli* K12 (K12/pCC1BAC-PrhtC-rhtC(mt)) strains transformed with the mutant library, and sequencing was performed to determine the mutation rate and the presence/absence of mutations at various locations, and as a result, it was found that the mutation rate was 1.2 kb⁻¹ under the case #1 condition and 2.0 kb⁻¹ under the case #2 condition. Both cases #1 and #2 were determined to satisfy the mutation rate suitable for securing a mutant library, and thus effective mutation selection was performed using the library prepared under the conditions described above.

Additionally, in order to prepare a pCC1BAC-PrhtC-rhtC(WT) vector as a control group, nucleotide sequence fragments (SEQ ID NO: 2) encoding RhtC WT were obtained from the genomic DNA of *E. coli* W3110 by performing PCR through a conventional method using a primer pair of SEQ ID NO: 9 and SEQ ID NO: 10. The PCR was performed as follows: denaturation at 95°C for 30 seconds; 25 cycles of denaturation at 95°C for 30 seconds annealing at 55°C for 30 seconds, and polymerization at 68°C for 30 seconds; and polymerization at 68°C for 60 seconds. The thus-obtained products were treated with *Dpn*I, and then cloned into the pCC1BAC-PrhtC digested with *Bam*HI restriction enzyme by the Gibson assembly method to obtain a library of recombinant mutant plasmid pCC1BAC-PrhtC_rhtC(WT) vector.

### Example 2: Confirmation of mutations in L-threonine efflux protein through library screening

After dispensing 300 µL of M9 minimal media containing 60 g/L L-threonine onto each well of a 96-deep-well plate, the colonies of K12/pCC1BAC-PrhtC-rhtC(mt), which was transformed with the mutant library prepared in Example 1, and the colonies of the control group (K12/pCC1BAC and K12/pCC1BAC-PrhtC-rhtC(WT)) were inoculated. The cells were cultured under the conditions of 1,200 rpm/15 hr/37°C, followed by OD measurement at a wavelength of 600 nm. The strains which did not exhibit growth inhibition in M9 minimal media containing 60 g/L of L-threonine, unlike wild-type *E. coli* K12 strains, were selected.

As a result, it was observed that most of the mutant strains showed almost no growth in the deep-well plate as in the control group (K12/pCC1BAC and K12/pCC1BAC-PrhtC-rhtC(WT)), and about 4,000 strains were selected from the primary screening based on the results of OD measurement. Among them, the top 5% (150 colonies) were subjected to secondary screening, and the colonies showing high average values were selected in triplicate. After excluding colonies with significant deviation, six strains in which growth was observed in contrast to the control strains were finally selected and named as K12/pCC1BAC-PrhtC_rhtC (m1 to m6), and the OD was recorded and shown in Table 4 below.

**[Table 4]**

| Strain Name | OD |
|---|---|
| K12/pCC1BAC | 0.14 |
| K12/pCC1BAC-PrhtC_rhtC(WT) | 0.17 |
| K12/pCC1BAC-PrhtC_rhtC(m1) | 1.21 |
| K12/pCC1BAC-PrhtC_rhtC(m2) | 1.16 |
| K12/pCC1BAC-PrhtC_rhtC(m3) | 1.25 |
| K12/pCC1BAC-PrhtC_rhtC(m4) | 1.26 |
| K12/pCC1BAC-PrhtC_rhtC(m5) | 1.49 |
| K12/pCC1BAC-PrhtC_rhtC(m6) | 1.31 |

In order to confirm the nucleotide sequence of the *rhtC* gene included in the selected six strains K12/pCC1BAC-PrhtC_rhtC (m1 to m6), DNA fragments including the *rhtC* gene were amplified by performing PCR under the same conditions as in Example 1 above using a primer pair of SEQ ID NO: 9 and SEQ ID NO: 10.

As a result of analyzing the nucleotide sequences of the amplified gene, it was confirmed that in the K12/pCC1BAC-PrhtC-rhtC (m1 to m6) strain, 1 or 2 mutations were introduced into a nucleotide sequence located between the 397th and the 399th bases downstream from the ORF start codon of the *rhtC* gene. That is, it was confirmed that in the K12/pCC1BAC-PrhtC-rhtC (m1 to m6) strain, the nucleotide sequence located between the 427th to the 429th bases was mutated from GGC to AGC or AGT based on SEQ ID NO: 2, thus including a sequence encoding a mutant RhtC (G133S) protein, which was in such a form that glycine (G) (*i.e*., the 133rd amino acid from the N-terminus of SEQ ID NO: 1) was substituted with serine (S).

### Example 3: Preparation of plasmid for expression of L-threonine efflux protein variants

In order to introduce a mutant gene for the expression of the L-threonine efflux protein variants confirmed in Example 2 above onto the chromosome of a strain of the genus *Corynebacterium,* the nucleotide sequence of the *rhtC* gene included in the selected four strains, a recombinant vector was prepared in the following manner.

First, in order to prepare an Ncg!1762-deleting vector, the nucleotide sequence fragments corresponding to the homologous upstream and downstream regions of Ncgl1762 were amplified by performing PCR under the same conditions as in Example 1 above using the genomic DNA of *Corynebacterium glutamicum* ATCC13032 strain as a template and using a primer pair of SEQ ID NO: 11 and SEQ ID NO: 12 and a primer pair of SEQ ID NO: 13 and SEQ ID NO: 14. Then, the amplified product was treated with Smal restriction enzyme, ligated with the pDCM2 vector (Korean Patent No. 2278000) heat-treated at 65°C for 20 minutes using an infusion cloning kit, and the resultant was transformed into *E*. *coli* DH5α. The transformed strain was plated on LB solid medium containing kanamycin (25 mg/L), and the selected colonies were cultured to obtain a plasmid, which was named pDCM2-ΔNcgl1762.

The primers used are shown in Table 5 below.

**[Table 5]**

| SEQ ID NO | Sequence Name | Sequence (5' -> 3') |
|---|---|---|
| 11 | 1762A F | TGAATTCGAGCTCGGTACCCGTGGCCAGTATTAATG GGCT |
| 12 | 1762A R | GTCTCAAGCAGAGActcgagCACCCTCAATGGGTCAG GCC |
| 13 | 1762B F | ACCCATTGAGGGTGctcgagTCTCTGCTTGAGACTTAA GC |
| 14 | 1762B R | GTCGACTCTAGAGGATCCCCAGGCTCTCTATGGGCA CCTC |

Then, vectors for inserting the wild-type *rhtC* and mutant *rhtC* genes at the Ncgl1762 site were prepared. The *rhtC*(WT) fragment was obtained from the genomic DNA of *E*. *coli* W3110 strain by performing PCR using a primer pair of SEQ ID NO: 15 and SEQ ID NO: 16 under the same conditions as in Example 1 above. Further, the mutant *rhtC*(G133S) fragment was obtained by amplification using, as a template, the *rhtC* fragment including a sequence encoding the mutant protein, in which glycine (*i.e*., the 133rd amino acid) was substituted with serine as confirmed in Example 2, using a primer pair of SEQ ID NO: 15 and SEQ ID NO: 16. Then, each fragment and the pDCM2-ΔNcgl1762 vector were treated with *Xho*I restriction enzyme, heat-treated at 65°C for 20 minutes, and ligated using an infusion cloning kit, and the resultant was transformed into *E. coli* DH5α. The strain was plated on LB solid medium containing kanamycin (25 mg/L), the selected colonies were cultured to obtain plasmids, and the thus-obtained plasmids were each named pDCM2-ΔNcgl1762::PrhtC-rhtC(WT) and pDCM2-ΔNcgl1762::PrhtC-rhtC(G133S).

The primers used are shown in Table 6 below.

**[Table 6]**

| SEQ ID NO | Sequence Name | Sequence (5' -> 3') |
|---|---|---|
| 15 | rhtC(m) F | GCCTGACCCATTGAGGGTGcGCCATGAACATTGGGT GAGC |
| 16 | rhtC(m) R | ATAACGCTTAAGTCTCAAGCAGAGActcgaTCACCGCG AAATAATCAAAT |

### Example 4: Preparation of Corynebacterium glutamicum KCCM12502P strain introduced with L-threonine efflux protein variant and analysis of L-threonine productivity

The pDCM2-ΔNcgl1762, pDCM2-ΔNcgl1762::PrhtC_rhtC(WT), and pDCM2-ΔNcgl1762::PrhtC_rhtC(G133S) vectors prepared in Example 3 above were each transformed into *Corynebacterium glutamicum* CA09-0903 strain (Accession No. KCCM12502P, KR Patent No. 10-2126951) by electroporation, and then subjected to secondary crossover to obtain an Ncgl1762-deficient strain and strains in which the wild-type *rhtC* or mutant *rhtC* gene was inserted at the Ncgl1762 position. The corresponding genetic manipulation was confirmed through genome sequencing and PCR using the primer pairs of SEQ ID NOS: 17 to 23, which are each capable of amplifying the external sites of the upstream and downstream regions of homologous recombination where the corresponding gene was inserted.

The primers used are shown in Table 7 below.

**[Table 7]**

| SEQ ID NO | Sequence Name | Sequence (5' -> 3') |
|---|---|---|
| 17 | pDC F | CTATTACGCCAGCTGGCGAAAG |
| 18 | pDC R | ATGTTGTGTGGAATTGTGAG |
| 19 | seq 1 | TTCTCTTGTTCCTTTCTGAG |
| 20 | seq 2 | TTCTGAGTAGCTGAGCTTCG |
| 21 | seq 3 | CCAGATGCTACGTGGTGCAC |
| 22 | seq 4 | TGGTCTGCTCGCTTCTCGAT |
| 23 | seq 5 | TATCTTCGTAGAGCACTGCT |

The thus-obtained Ncgl1762-deficient strain and the strains in which wild-type *rhtC* or mutant *rhtC* gene was inserted at the Ncgl1762 position were named 12502P-del, 12502P-WT, and 12502P-G133S, respectively.

Flask culture was performed using the following medium to analyze L-threonine productivity for the three mutants prepared above. The parent strain, *Corynebacterium glutamicum* KCCM12502P strain, was used as the control, and the amount of L-threonine production was measured by culturing in the following manner.

First, each strain was inoculated into a 250 mL corner-baffle flask containing 25 mL of seed medium, and the flask was incubated at 30°C for 20 hours with shaking at 200 rpm. Then, 1 mL of the seed culture was inoculated into a 250 mL corner-baffle flask containing 24 mL of production medium and the flask was incubated at 32°C for 72 hours with shaking at 200 rpm. The compositions of the seed medium and the production medium are each shown below, and the concentrations of L-threonine produced according to the culture are shown in Table 8.

### <Seed Medium (pH 7.0)>

glucose 20 g, peptone 10 g, yeast extract 5 g, urea 1.5 g, KH₂PO₄ 4 g, K₂HPO₄ 8 g, MgSO₄·7H₂O 0.5 g, biotin 0.1 mg, thiamine HCl 1 mg, calcium pantothenate 22 mg, nicotinamide 2 mg (based on 1 L of distilled water)

### <Production Medium (pH 7.0)>

glucose 63 g, (NH₄)₂SO₄ 28 g, soybean protein 20 g, molasses 14 g, KH₂PO₄ 1.1 g, MgSO₄·7H₂O 1.2 g, biotin 1.8 mg, thiamine HCl 9 mg, calcium pantothenate 9 mg, MnSO₄ 180 mg, FeSO₄ 200 mg, ZnSO₄ 1 mg, CuSO₄ 1 mg, CaCOs 30 g (based on 1 L of distilled water)

**[Table 8]**

| Strain Name | L-Threonine (g/L) | | | |
|---|---|---|---|---|
| | Batch 1 | Batch 2 | Batch 3 | Average |
| KCCM12502P | 3.31 | 3.27 | 3.21 | 3.26 |
| KCCM12502P-del | 3.26 | 3.34 | 3.22 | 3.27 |
| KCCM12502P-WT | 3.41 | 3.45 | 3.39 | 3.42 |
| KCCM12502P-G133S | 4.32 | 4.36 | 4.41 | 4.36 |

As a result, as shown in Table 8, it was confirmed that the control group KCCM12502P produced 3.26 g/L of L-threonine; KCCM12502P-del produced 3.27 g/L of L-threonine; and KCCM12502P-G133S, introduced with the mutant rhtC(G133S), produced 4.36 g/L of L-threonine.

With regard to fermentation yield of L-threonine, KCCM12502P-WT showed a 5% increase compared to KCCM12502P, whereas KCCM12502P-G133S strain introduced with a mutant rhtC(G133S) showed a 33% increase.

### Example 5: Preparation of Corynebacterium glutamicum KCCM11222P strain introduced with L-threonine efflux protein variant and analysis of L-threonine productivity

In order to confirm whether L-threonine productivity is also increased by introducing mutant RhtC in other *Corynebacterium glutamicum* strains that produce L-threonine, the pDCM2-ΔNcgl1762, pDCM2-ΔNcgl1762::PrhtC_rhtC(WT), and pDCM2-ΔNcgl1762::PrhtC_rhtC(G133S) vectors prepared in Example 3 above were each transformed into *Corynebacterium glutamicum* KFCC10881-THR (Accession No. KCCM11222P, US 10590446 B2) in the same manner as in Example 4 above, and the resulting transformants were named 11222P-del, 11222P-WT, and 11222P-G133S, respectively.

In order to analyze the L-threonine productivity of the three mutant strains prepared, flask culture was performed using the following media. The parent strain *Corynebacterium glutamicum* KCCM11222P was used as the control, and L-threonine productivity was measured by culturing in the following manner.

First, each strain was inoculated into a 250 mL corner-baffle flask containing 25 mL of seed medium, and the flask was incubated at 30°C for 20 hours with shaking at 200 rpm. Then, 1 mL of the seed culture was inoculated into a 250 mL corner-baffle flask containing 24 mL of production medium, and the flask was incubated at 32°C for 72 hours with shaking at 200 rpm. The compositions of the seed medium and the production medium are each shown below, and the concentrations of L-threonine produced according to the culture are shown in Table 9.

### <Seed Medium (pH 7.0)>

glucose 20 g, peptone 10 g, yeast extract 5 g, urea 1.5 g, KH₂PO₄ 4 g, K₂HPO₄ 8g, MgSO₄·7H₂O 0.5 g, biotin 0.1 mg, thiamine HCl 1 mg, calcium pantothenate 22 mg, nicotinamide 2 mg (based on 1 L of distilled water)

### <Production Medium (pH 7.0)>

glucose 63 g, (NH₄)₂SO₄ 28 g, soybean protein 20 g, molasses 14 g, KH₂PO₄ 1.1 g, MgSO₄·7H₂O 1.2 g, biotin 1.8 mg, thiamine HCl 9 mg, calcium pantothenate 9 mg, MnSO₄ 180 mg, FeSO₄ 200 mg, ZnSO₄ 1 mg, CuSO₄ 1 mg, CaCOs 30 g (based on 1 L of distilled water)

**[Table 9]**

| Strain Name | L-Threonine (g/L) | | | |
|---|---|---|---|---|
| | Batch 1 | Batch 2 | Batch 3 | Average |
| KCCM11222P | 7.11 | 7.14 | 7.11 | 7.12 |
| KCCM11222P-del | 7.21 | 7.18 | 7.16 | 7.18 |
| KCCM11222P-WT | 7.34 | 7.38 | 7.42 | 7.38 |
| KCCM11222P-G133S | 8.94 | 8.89 | 9.01 | 8.95 |

As a result, as shown in Table 9, it was confirmed that the L-threonine-producing *Corynebacterium glutamicum* KCCM11222P strain, into which a mutant rhtC(G133S) was inserted, showed an average increase of 25% in L-threonine productivity.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. An L-threonine efflux protein variant in which the amino acid corresponding to the 133rd position of SEQ ID NO: 1 is substituted with another amino acid.

2. The L-threonine efflux protein variant according to claim 1, wherein the other amino acid is selected from the group consisting of serine, threonine, cysteine, tyrosine, asparagine, and glutamine.

3. The L-threonine efflux protein variant according to claim 2, wherein the other amino acid is serine.

4. The L-threonine efflux protein variant according to claim 1, wherein the L-threonine efflux protein variant has sequence identity of at least 70% and less than 100% to the amino acid sequence of SEQ ID NO: 1.

5. A polynucleotide encoding the L-threonine efflux protein variant according to any one of claims 1 to 4.

6. A microorganism, which comprises an L-threonine efflux protein variant in which the amino acid corresponding to the 133rd position of SEQ ID NO: 1 is substituted with another amino acid; or a polynucleotide encoding the same.

7. The microorganism according to claim 6, wherein the microorganism has increased L-threonine productivity compared to a microorganism of the genus *Corynebacterium* comprising a wild-type L-threonine efflux protein having an amino acid sequence of SEQ ID NO: 1 or a polynucleotide encoding the same.

8. The microorganism according to claim 6, wherein the microorganism is a microorganism of the genus *Corynebacterium.*

9. The microorganism according to claim 8, wherein the microorganism of the genus *Corynebacterium* is *Corynebacterium glutamicum.*

10. A method for producing L-threonine, comprising culturing a microorganism which comprises an L-threonine efflux protein variant in which the amino acid corresponding to the 133rd position of SEQ ID NO: 1 is substituted with another amino acid; or a polynucleotide encoding the same in a culture medium.

11. A composition for producing L-threonine, comprising a L-threonine efflux protein variant in which the amino acid corresponding to the 133rd position of SEQ ID NO: 1 is substituted with another amino acid; a polynucleotide encoding the same; a vector comprising the polynucleotide; a microorganism comprising the L-threonine efflux protein variant or the polynucleotide encoding the same; a culture in which the microorganism has been cultured; or a combination of two or more thereof.

12. Use of a L-threonine efflux protein variant in which the amino acid corresponding to the 133rd position of SEQ ID NO: 1 is substituted with another amino acid; a polynucleotide encoding the same; a vector including the polynucleotide; or a microorganism comprising the L-threonine efflux protein variant or the polynucleotide encoding the same, for producing L-threonine.
